(19) Europäisches Patentamt
European Patent Office
Office européen des brevets

(11) Veröffentlichungsnummer: **0 101 407**
**B1**

(12) # EUROPÄISCHE PATENTSCHRIFT

(45) Veröffentlichungstag der Patentschrift: **21.09.88**

(21) Anmeldenummer: **83810318.2**

(22) Anmeldetag: **13.07.83**

(51) Int. Cl.⁴: **C 07 D 405/12,** C 07 D 409/12, C 07 D 403/12, C 07 D 401/12, C 07 D 239/42, C 07 D 239/52, C 07 D 239/46, C 07 D 239/48, C 07 D 251/16, C 07 D 251/44, C 07 D 251/26 // C07D333/38, C07C143/83, C07C155/02

(54) **Verfahren zur Herstellung von herbiziden und pflanzenwuchsregulierenden Sulfonylharnstoffen und neue Sulfonylcarbamate als Zwischenprodukte.**

(30) Priorität: **19.07.82 CH 4396/82**

(43) Veröffentlichungstag der Anmeldung: **22.02.84 Patentblatt 84/8**

(45) Bekanntmachung des Hinweises auf die Patenterteilung: **21.09.88 Patentblatt 88/38**

(84) Benannte Vertragsstaaten: **CH DE FR GB LI**

(56) Entgegenhaltungen:
**EP-A-0 041 404**
**EP-A-0 044 807**
**EP-A-0 044 809**
**EP-A-0 087 780**
**US-A-3 621 057**
**US-A-3 644 634**
**US-A-3 752 851**
**US-A-3 796 719**
**US-A-3 803 176**
**US-A-3 825 665**
**US-A-3 849 110**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**
Erfinder: **Föry, Werner, Dr., Benkenstrasse 65, CH- 4054 Basel (CH)**
Erfinder: **Töpfl, Werner, Dr., Dorneckstrasse 68, CH- 4143 Dornach (CH)**

EP 0 101 407 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur Herstellung von herbiziden und pflanzenwuchsregulierenden Sulfonylharnstoffen sowie neue als Zwischenprodukte hergestellte Sulfonylcarbamate.

Die nach dem neuen erfindungsgemässen Verfahren herzustellenden Sulfonylharnstoffe sind in den US-Patenten 4 127 405, 4 301 286 und 4,302,241, den europäischen Offenlegungsschriften 23422, 44807 und 44808 sowie den schweizerischen Patentanmeldungen 4667/81-0, 5075/81-2 und 2205/82-3 mit ihren Eigenschaften beschrieben.

Die bekannten Verfahren sind insofern nachteilig, als entweder mit Isocyanat- oder Isothiocyanat-Derivaten gearbeitet werden muss deren Handhabung wegen der grossen Reaktivität dieser Verbindungsklasse schwierig ist, oder bei der Synthese aus Phenylcarbamaten ökologisch belastende Nebenprodukte wie z. B. Phenole anfallen.

Es ist somit Ziel der vorliegenden Erfindung, ein Verfahren bereitzustellen, dass die Verwendung schlecht handhabbarer Verbindungen und den Anfall von umweltbelastenden Nebenprodukten vermeidet.

Erfindungsgemäss wird somit vorgeschlagen, die herbiziden und pflanzenregulierenden Sulfonylharnstoffe der allgemeinen Formel I

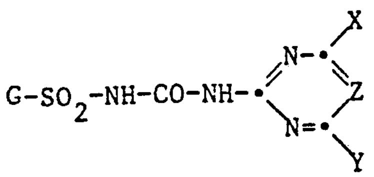 (I)

worin

Z einen -N= oder -CH= -Rest,

G einen Rest der Formeln

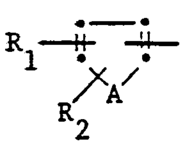 oder 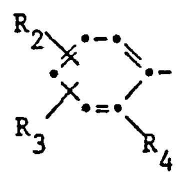 ,

X $C_1$-$C_4$-Alkyl, $C_1$-$C_4$-Halogenalkyl, $C_1$-$C_4$-Alkoxy, $C_1$-$C_4$-Alkylthio, Halogen, $C_1$-$C_4$-Halogenalkoxy, $C_1$-$C_4$-Alkylamino oder Di-$C_1$-$C_4$-alkylamino,

Y $C_1$-$C_4$-Alkyl, $C_1$-$C_4$-Alkoxy oder $C_1$-$C_4$-Halogenalkoxy bedeuten, worin

A für Sauerstoff, Schwefel, -$NR_5$- oder -C=N-, wobei $R_5$ Wasserstoff, $C_1$-$C_4$-Alkyl oder -CO-$C_1$-$C_4$-Alkyl bedeutet,

$R_1$ für Wasserstoff, $C_1$-$C_4$-Alkyl, Halogen, Nitro, Cyan, -$NH_2$, -$S(O)_n$-$C_1$-$C_4$-Alkyl, -$SO_2$-$C_1$-$C_4$-Alkoxy, -$SO_2$-di-$C_1$-$C_4$-alkylamino, -CHO, -$CONH_2$, -D-$C_3$-$C_5$-Alkinyl -CO-D-$C_3$-$C_5$-Alkinyl, -D-$C_1$-$C_4$-Alkyl, -D-$C_3$-$C_5$-Alkenyl, -CO-$C_1$-$C_4$-Alkyl, -CO-D-$C_1$-$C_4$-Alkyl oder -CO-D-$C_3$-$C_5$-Alkenyl, wobei n eines oder zwei und D eine Sauerstoff-, Schwefel-, -NH- oder -N($C_1$-$C_4$-Alkyl)-Brücke bedeuten,

$R_2$ für Wasserstoff, Halogen, $CF_3$, $NO_2$, $C_1$-$C_4$-Alkyl oder $C_1$-$C_4$-Alkoxy und

$R_3$ für Wasserstoff, Fluor oder Chlor stehen und

$R_4$ für Halogen, $C_1$-$C_4$-Alkyl, Nitro, Cyan, -$NH_2$, -$S(O)_n$-$C_1$-$C_4$-Alkyl, -$SO_2$-$C_1$-$C_4$-Alkoxy, -$SO_2$-di-$C_1$-$C_4$-alkylamino, -CHO, -$CONH_2$ -D-$C_3$-$C_5$-Alkinyl, -CO-D-$C_3$-$C_5$-Alkinyl, Trifluormethyl oder durch Cyan, Halogen, $C_1$-$C_4$-Alkoxy oder $C_1$-$C_4$-Alkylthio substituiertes $C_1$-$C_4$-Alkoxy, -D-$C_1$-$C_4$-Alkyl, -D-$C_3$-$C_5$-Alkenyl, -CO-$C_1$-$C_4$-Alkyl, 1,2-Dichlorvinyloxy, -CO-D-$C_1$-$C_4$-Alkyl oder -CO-D-$C_3$-$C_5$-Alkenyl,

wobei n eines oder zwei und D eine Sauerstoff-, Schwefel-, -NH- oder -N($C_1$-$C_4$-Alkyl)-Brücke bedeuten, steht,

in der Weise herzustellen, dass man ein Sulfonamid der Formel II

$G$-$SO_2$-$NH_2$ (II)

in Gegenwart einer Base mit einem Chlorameisensäureester der Formel III

Cl-CO-Q-T (III)

oder dass man ein Sulfonylchlorid der Formel IV

$G\text{-}SO_2\text{-}Cl$ (IV)

in Gegenwart einer Base mit einem Urethan der Formel V

$H_2N\text{-}CO\text{-}Q\text{-}T$ (V)

worin G die unter Formel I gegebene Bedeutung hat, und
Q Sauerstoff oder Schwefel und
T $C_1$-$C_4$-Alkyl, Benzyl $C_1$-$C_4$-Halogenalkyl, $C_1$-$C_3$-Cyanalkyl, $C_2$-$C_5$-Alkenyl, oder Alkoxyalkyl oder Alkylthioalkyl mit insgesamt höchstens 5 Kohlenstoffatomen bedeuten, umsetzt und das entstandene Carbamat der Formel VI

$G\text{-}SO_2\text{-}NH\text{-}CO\text{-}Q\text{-}T$ (VI)

durch Umsetzung mit einem Amin der Formel VII

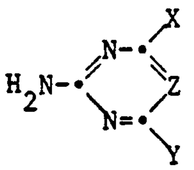

(VII)

worin G, Q, T, X, Y und Z die oben gegebenen Bedeutungen haben, in den Sulfonylharnstoff der Formel 1 überführt.

Unter Halogen ist im Rahmen der obigen Definition im allgemeinen Fluor, Chlor, Brom oder Jod zu verstehen. Bevorzugt sind Fluor und Chlor. Als Substituent des Restes G ist insbesondere Chlor und als Substituent einer Alkylgruppe, die ihrerseits Teil eines Restes sein kann, ist insbesondere Fluor bevorzugt.

Beispiele für Alkyl sind Methyl, Äthyl, n-Propyl, i-Propyl oder die isomeren Butyl. Alkyl ist dabei selbst als Substituent oder als Teil eines anderen Substituenten wie beispielsweise Alkoxy oder Alkylthio zu verstehen. Bevorzugte Alkylreste sind jeweils unverzweigte Alkylketten, insbesondere aber Methyl und Äthyl.

In der Regel sind unter Alkenyl, Allyl, 2-Butenyl, 3-Butenyl, 2-Isobutenyl, Isopropenyl, 2-Pentenyl, 3-Pentenyl, und 4-Pentenyl, insbesondere aber Allyl und 4-Pentenyl zu verstehen.

Unter Alkinyl versteht man im allgemeinen Propargyl, 2-Butinyl, 3-Butinyl, Methylpropargyl, 2-Pentinyl, 3-Pentinyl und 4-Pentinyl.

Die Heterocyclen, die unter die Definition des Restes G fallen, sind Thiophen, Furan, Pyrrol und Pyridin.

Unter den Pyrimidin- und Triazinringen sind diejenigen bevorzugt, in denen X Methyl, Äthyl, Fluormethyl, Trifluormethyl, Methoxy, Äthoxy, i-Propyloxy, Methylthio, Chlor, Brom, Difluormethoxy, 2,2,2-Trifluoräthoxy, Methylamino oder Dimethylamino und Y Methyl, Methoxy oder Difluormethoxy bedeuten.

Die Umsetzung der Sulfonamide der Formel II mit den Chlorameisensäureestern der Formel III kann in Gegenwart eines geeigneten inerten aprotischen Lösungsmittels oder auch in Abwesenheit eines Lösungsmittels durchgeführt werden. Als vorteilhaft hat sich jedoch die Verwendung eines Lösungsmittel erwiesen. Geeignete Lösungsmittel sind Kohlenwasserstoffe wie Benzol, Toluol und Xylol; Äther wie Diäthyläther, Äthylenglykoldimethyläther, Diäthylenglykoldimethyläther, Tetrahydrofuran und Dioxan; Ketone wie Aceton, Äthylmethylketon und Cyclohexanon; Nitrile wie Acetonitril und Propionitril; und Dimethylsulfoxid. Die Umsetzung erfolgt in Gegenwart von mindestens äquimolaren Mengen einer Base. Geeignete Basen sind Carbonate wie Natrium- und Kaliumcarbonat, Hydrogencarbonate wie Natrium- und Kaliumhydrogencarbonat, Oxide wie Calcium- und Magnesiumoxid und tertiäre Amine wie Trimethylamin, Triäthylamin, Chinuclidin, Chinolin, Pyridin und Tripropylamin. Mit Vorteil wird die Base im Überschuss eingesetzt. So werden pro Mol Sulfonamid vorzugsweise 1 bis 5 Mol, insbesondere 1,1 bis 1,5 Mol eingesetzt. Grössere Basenüberschüsse werden besonders dann verwendet, wenn die Reaktion ohne Lösungsmittel durchgeführt wird und die Base, vorzugsweise ein flüssiges tertiäres Amin, gleichzeitig als Reaktionsmedium dient. Die Reaktionstemperaturen liegen in der Regel zwischen 0°C und 140°C, vorzugsweise zwischen 10° und 80°C.

Die Umsetzung der Sulfonylchloride der Formel IV mit den Urethanen der Formel V wird unter den gleichen Reaktionsbedingungen durchgeführt wie die Reaktion der Verbindungen der Formeln II und III.

Die als Zwischenprodukte erhaltenen Sulfonylcarbamate der Formel VI werden mit den Aminen der Formel VII zur Reaktion gebracht, indem das Gemisch beider Reaktionspartner erhitzt wird, bis eine Abspaltung des Alkohols oder Mercaptans eintritt. Die Umsetzung kann dabei sowohl ohne Lösungsmittel, als auch in Gegenwart eines inerten Lösungsmittels erfolgen. Im allgemeinen wird das Reaktionsgemisch bis zum Eintritt der Abspaltungsreaktion von Alkohol oder Mercaptan erwärmt und bei dieser Temperatur gehalten bis ein vollständiger Umsatz erreicht ist. Die Temperetur beträgt dabei in der Regel 50° bis 220°C, vorzugsweise 80° bis 160°C. Als geeignete Lösungsmittel haben sich bewährt: Kohlenwasserstoffe wie Benzol, Toluol, Xylol, Mesitylen, Tetrahydronaphthalin, Decalin, Cyclohexan und höhersiedende Benzinfraktionen; Äther wie Tetrahydrofuran, Dioxan, Äthylenglykoldimethyläther, Diäthylenglykoldimethyläther und Diphenyläther, Nitrile wie Acetonitril oder Propionitril, Ketone wie Äthylmethylketon, Cyclohexanon und Aceton und Dimethylsulfoxid.

In einer bevorzugten Ausführungsform des erfindungsgemässen Verfahrens wird daher in der Weise vorgegangen, dass man ein Sulfonamid der Formel II mit einem Chlorameisensäureester der Formel III, oder dass man ein Sulfonylchlorid der Formel IV in Gegenwart einer Base in einem inerten Lösungsmittel bei 10° bis 80°C mit einem Urethan umsetzt und das erhaltene Carbamat der Formel VI zusammen mit einem Amin der Formel VII in einem inerten Lösungsmittel auf 80° bis 160°C erhitzt bis die Abspaltungsreaktion von Alkohol oder Mercaptan abgeschlossen ist und das Produkt isoliert.

Die Ausgangsverbindungen der Formeln II, III, IV, V und VII sind bekannt oder können nach bekannten Methoden hergestellt werden.

Die Sulfonylcarbamate der engeren Unterformel VIa sind neu und wurden speziell zur für die Durchführung des erfindungsgemässen Verfahrens entwickelt. Sie bilden deshalb einen weiteren Gegenstand der vorliegenden Erfindung.

Die neuen Sulfonylcarbamate entsprechend der allgemeinen Formel VIa

$$C\text{-}SO_2\text{-}NH\text{-}CO\text{-}Q\text{-}T \qquad (VIa)$$

worin

Q Sauerstoff oder Schwefel,

T $C_1$-$C_4$-Alkyl, $C_1$-$C_4$-Halogenalkyl, $C_1$-$C_3$-Cyanalkyl, $C_2$-$C_5$-Alkenyl, Benzyl oder Alkoxyalkyl oder Alkylthioalkyl mit insgesamt höchstens 5 Kohlenstoffatomen und

G einen Rest der Formeln

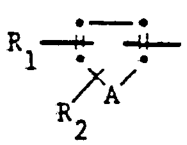 oder 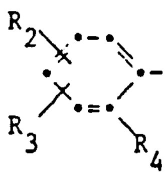 ,

bedeuten, worin

A für Sauerstoff, Schwefel, $-NR_5-$ oder $-C=N-$, wobei $R_5$ Wasserstoff, $C_1$-$C_4$-Alkyl oder -CO-$C_1$-$C_4$-Alkyl bedeutet,

$R_1$ für Wasserstoff, $C_1$-$C_4$-Alkyl, Halogen, Nitro, Cyan, $-NH_2$, $-S(O)_n$-$C_1$-$C_4$-Alkyl, $-SO_2$-$C_1$-$C_4$-Alkoxy, $-SO_2$-di-$C_1$-$C_4$-alkylamino, -CHO, $-CONH_2$, -D-$C_3$-$C_5$-Alkinyl -CO-D-$C_3$-$C_5$-Alkinyl, -D-$C_1$-$C_4$-Alkyl, -D-$C_3$-$C_5$-Alkenyl, -CO-$C_1$-$C_4$-Alkyl, -CO-D-$C_1$-$C_4$-Alkyl oder -CO-D-$C_3$-$C_5$-Alkenyl, wobei n eines oder zwei und D eine Sauerstoff-, Schwefel-, -NH- oder -N($C_1$-$C_4$-Alkyl)-Brücke bedeuten, und

$R_2$ für Wasserstoff, Halogen, $CF_3$, $NO_2$, $C_1$-$C_4$-Alkyl oder $C_1$-$C_4$-Alkoxy und

$R_3$ für Wasserstoff, Fluor oder Chlor stehen und

$R_4$ für Halogen, $C_1$-$C_4$-Alkyl, Nitro, Cyan, $-NH_2$, $-S(O)_n$-$C_1$-$C_4$-Alkyl, $-SO_2$-$C_1$-$C_4$-Alkoxy $-SO_2$-di-$C_1$-$C_4$-alkylamino, -CHO, $-CONH_2$, -D-$C_3$-$C_5$-Alkinyl -CO-D-$C_3$-$C_5$-Alkinyl Trifluormethyl oder durch Cyan, Halogen, $C_1$-$C_4$-Alkoxy oder $C_1$-$C_4$-Alkylthio substituiertes $C_1$-$C_4$-Alkoxy, -D-$C_1$-$C_4$-Alkyl, -D-$C_3$-$C_5$-Alkenyl, -CO-$C_1$-$C_4$-Alkyl, 1,2-Dichlorvinyloxy, -CO-D-$C_1$-$C_4$-Alkyl oder -CO-D-$C_3$-$C_5$-Alkenyl, wobei n eines oder zwei und D eine Sauerstoff-, Schwefel-, -NH- oder -N($C_1$-$C_4$-Alkyl)-Brücke bedeuten, steht, mit der Massgabe, dass

a) der Rest -Q-T nicht Äthoxy oder Butyloxy bedeutet wenn G für den unsubstituierten 2-Thienylrest steht,

b) der Rest -Q-T nicht Methylthio bedeutet, wenn G für den 2-Tolylrest steht und

c) der Rest -Q-T nicht Äthoxy bedeutet, wenn C für den 2-Trifluor-methylphenylrest oder den 2-Anisylrest steht,

d) der Rest -Q-T nicht Methoxy bedeutet, wenn G für den 2-Methoxy-carbonylphenylrest oder den 2-Äthylthiocarbonylphenylrest steht.

Die folgenden Beispiele dienen der näheren Erläuterung der Erfindung.

**Beispiel 1: N-(2-Difluormethoxyphenyl-sulfonyl)-äthoxycarbamat**

Einer Lösung von 2,2 g Carbamidsäureäthylester in 30 ml Äthylenglykol-dimethyläther werden bei 20° bis 25°C 1,15 g 55-%-iges Natriumhydrid zugesetzt. Die entstehende Suspension wird 30 Minuten bei 20° bis 25°C gerührt, auf 10°C abgekühlt und tropfenweise mit 6,0 g 2-Difluormethoxy-benzolsulfonylchlorid versetzt. Die Reaktion ist exotherm. Es findet eine schwache Gasentwicklung statt. Nachdem das Reaktionsgemisch für eine Stunde bei 20° bis 25°C gerührt worden ist, wird das Lösungsmittel abgedampft und der Rückstand mit 50 ml Eiswasser versetzt. Durch Extraktion mit Äthylacetat, Trocknen und Eindampfen der organischen Phase und säulenchromatographische Reinigung des Rückstandes an Kieselgel mit Methylenchlorid erhält man 1,7 g (23,3 % d. Th.) N-(2-Difluormethoxyphenyl-sulfonyl)-äthylcarbamat, Smp. 111 - 113°C.

**Beispiel 2: N-(2-Difluormethoxyphenyl-sulfonyl)-N'-(4-methoxy-6-methyl1,3,5-triazin-2-yl)-harnstoff.**

Eine Lösung von 1,48 g N-(2-Difluormethoxyphenyl-sulfonyl)-äthylcarbamat und 0,62 g 2-Amino-4-methoxy-6-methyl-1,3,5-triazin in 20 ml Chlorbenzol zum Rückfluss erhitzt.

Nach dem Eindampfen der gelben Lösung und chromatographischer Reinigung des Rückstandes erhält man ein farbloses Öl, das aus Aceton/Diäthyläther-Gemisch durch Zusatz von Hexan auskristallisiert. Man erhält 0,45 g (23,1 % d.Th.) N-(2-Difluormethoxyphenyl-sulfonyl)-N'-(4-methoxy-6-methyl-1,3,5-triazin-2-yl)-harnstoff, Smp. 140 - 141°C.

**Beispiel 3: N-(2-Chlorphenyl-sulfonyl)-äthylcarbamat.**

Ein Gemisch aus 12,25 g 2-Chlorphenylsulfonamid, 21,6 g wasserfreiem Kaliumcarbonat und 80 ml trockenem Aceton wird bei 20° bis 25°C mit 6,65 ml Chlorameisensäure-äthylester versetzt und für 2 Stunden zum Rückfluss erhitzt. Nach dem Abkühlen wird Reaktionsgemisch mit 300 ml Eiswasser aufgenommen, filtriert, mit konzentrierter Salzsäure bis zu einem pH-Wert von 2 angesäuert und mit Äthylacetat extrahiert.

Die vereinigten organischen Phasen werden mit Wasser gewaschen, über Natriumsulfat getrocknet und eingedampft. Durch Kristallisation aus Alkohol/Wassergemisch erhält man 14,9 g N-(2-Chlorphenyl-sulfonyl)-äthylcarbamat, Smp. 150 - 152°C.

**Beispiel 4: N-(2-Methoxycarbonylphenyl-sulfonyl)-N'-(4-difluormethoxy-6-methyl-pyrimidin-2-yl)-harnstoff.**

Eine Lösung von 2,73 g N-(2-Methoxycarbonylphenyl-sulfonyl)-methylcarbamat und 1,75 g 2-Amino-4-difluormethoxy-6-methyl-pyrimidin in 50 ml absolutem Dioxan wird für 24 Stunden am Rückfluss gekocht. Nach dem Abdampfen des Lösungsmittels wird der Rückstand in 5-%-iger Natriumcarbonat-Lösung gelöst, filtriert, mit konzentrierter Salzsäure bis zu einem pH-Wert von 2 angesäuert und mit Äthylacetat extrahiert. Durch Trocknen der vereinigten organischen Phasen über Natriumsulfat und Abdampfen des Lösungsmittels erhält man 3,2 g N-(2-Methoxycarbonylphenyl-sulfonyl)-N'-(4-difluormethoxy-6-methyl-pyrimidin-2-yl)-harnstoff, Smp. 162 - 164°C.

In analoger Weise werden die folgenden Zwischenprodukte der Formel VIa hergestellt.

**Tabelle 1:**

| Verb. Nr. | G | -Q-T | phys. Daten |
|---|---|---|---|
| 1 | 2-Cl-$C_6H_4$- | -$OC_2H_5$ | Smp. 150 - 152°C |
| 2 | 2-$OCH_3$-5-$OCH_3$-$C_6H_3$- | -$OC_2H_5$ | Smp. 132 - 133°C |
| 3 | 2-$OCH_3$-5-$OCH_3$-$C_6H_3$- | -$SCH_3$ | Smp. 182°C (Zers.) |
| 4 | 2-$OCHF_2$-$C_6H_4$- | -$OCH_3$ | Smp. 138 - 140°C |
| 5 | 2-$OCHF_2$-$C_6H_4$- | -$OC_2H_5$ | Smp. 107 - 111°C |
| 6 | 2-$OCHF_2$-$C_6H_4$- | -$OC_4H_9$-n | $n^{20}_D = 1.4958$ |
| 7 | 2-$OCH_3$-$C_6H_4$- | -$OCH_3$ | |
| 8 | 2-Cl-$C_6H_4$- | -$OCH_3$ | |
| 9 | 2-Cl-$C_6H_4$- | -O-$CH_2$-CH=$CH_2$ | |
| 10 | 2-Cl-$C_6H_4$- | -O-$CH_2$-$C_6H_5$ | |
| 11 | 2-Cl-$C_6H_4$- | -O-$CH_2$-$CF_3$ | |
| 12 | 2-Cl-$C_6H_4$- | -O-$CH_2$-$CCl_3$ | |
| 13 | 2-Cl-$C_6H_4$- | -S-$CH_3$ | |
| 14 | 2-$COOCH_3$-$C_6H_4$- | -$OCH_3$ | Smp. 162 - 164°C |
| 15 | 2-$NO_2$-$C_6H_4$- | -$OCH_3$ | |
| 16 | 2-[-O$(CH_2)_2$-$OCH_3$]-$C_6H_4$- | -$OCH_3$ | |
| 17 | 2-$CF_3$-$C_6H_4$- | -$OCH_3$ | |
| 18 | 2-$CH_3$-$C_6H_4$- | -$OCH_3$ | |
| 19 | 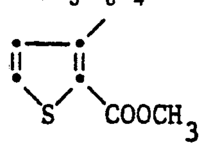 | -$OCH_3$ | |

| | | | |
|---|---|---|---|
| 20 | 2-$COOCH_3$-$C_6H_4$- | -$OC_2H_5$ | Smp. 73 - 75°C |
| 21 | 2-$COOCH_3$-$C_6H_4$- | -$OCH_2$-$CF_3$ | Smp. 106 - 108°C |
| 22 | 2-$COOCH_3$-$C_6H_4$- | -$OCH_2$-$C_6H_5$ | Smp. 108°C |
| 23 | 2-$COOCH_3$-$C_6H_4$- | -$OCH_2$-$CH=CH_2$ | Smp. 51 - 59°C |
| 24 | 2-$COOCH_3$-$C_6H_4$- | -$OCH_2CH_2OCH_3$ | $n^{21}_D$ - 1,5110 |
| 25 | 2-$COOCH_3$-$C_6H_4$- | -$OC_4H_9$(n) | Smp. 79 - 82°C |
| 26 | 2-$COOCH_3$-$C_6H_4$- | -$OCH_2CCl_2$ | $n^{21}_D$: 1,528 |
| 27 | 2-$COOCH_3$-$C_6H_4$- | -$OCH_2CH_2CN$ | Smp. 87°- 89°C |
| 28 | 2-$COOCH_3$-$C_6H_4$- | -$SCH_3$ | Smp. 133 - 4°C |
| 29 | 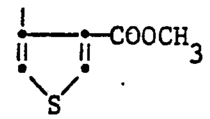 | -$OCH_3$ | |
| 30 | 2-$OCH_2CH_2Cl$-$C_6H_4$- | -$OCH_3$ | |

**Patentansprüche**

1. Verfahren zur Herstellung eines Sulfonylharnstoffes der allgemeinen Formel I

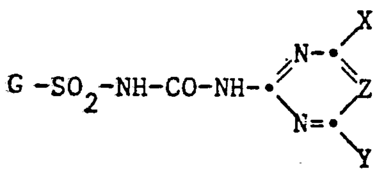

(I)

worin
Z einen -N= oder -CH= -Rest,
G einen Rest der Formeln

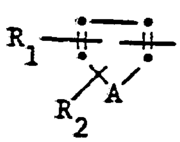

oder

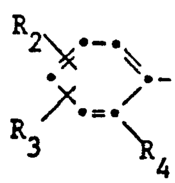

,

X $C_1$-$C_4$-Alkyl, $C_1$-$C_4$-Halogenalkyl, $C_1$-$C_4$-Alkoxy, $C_1$-$C_4$-Alkylthio, Halogen, $C_1$-$C_4$-Halogenalkoxy, $C_1$-$C_4$-Alkylamino oder Di-$C_1$-$C_4$-alkylamino,
Y $C_1$-$C_4$-Alkyl, $C_1$-$C_4$-Alkoxy oder $C_1$-$C_4$-Halogenalkoxy bedeuten, worin
A für Sauerstoff, Schwefel, -$NR_5$- oder -C=N-, wobei $R_5$ Wasserstoff, $C_1$-$C_4$-Alkyl oder -CO-$C_1$-$C_4$-Alkyl bedeutet
$R_1$ für Wasserstoff, $C_1$-$C_4$-Alkyl, Halogen, Nitro, Cyan, -$NH_2$, -$S(O)_n$-$C_1$-$C_4$-Alkyl, -$SO_2$-$C_1$-$C_4$-Alkoxy, -$SO_2$-di-$C_1$-$C_4$-alkylamino, -CHO, -$CONH_2$, -D-$C_3$-$C_5$-Alkinyl -CO-D-$C_3$-$C_5$-Alkinyl, -D-$C_1$-$C_4$-Alkyl, -D-$C_3$-$C_5$-Alkenyl, -CO-$C_1$-$C_4$-Alkyl, -CO-D-$C_1$-$C_4$-Alkyl oder -CO-D-$C_3$-$C_5$-Alkenyl, wobei n eines oder zwei und D eine Sauerstoff-, Schwefel-, -NH- oder -N($C_1$-$C_4$-Alkyl)-Brücke bedeuten,
$R_2$ für Wasserstoff, Halogen, $CF_3$, $NO_2$, $C_1$-$C_4$-Alkyl oder $C_1$-$C_4$-Alkoxy und
$R_3$ für Wasserstoff, Fluor oder Chlor stehen und
$R_4$ für Halogen, $C_1$-$C_4$-Alkyl, Nitro, Cyan, -$NH_2$, -$S(O)_n$-$C_1$-$C_4$-Alkyl, -$SO_2$-$C_1$-$C_4$-Alkoxy, -$SO_2$-di-$C_1$-$C_4$-alkylamino, -CHO, -$CONH_2$ -D-$C_3$-$C_5$-Alkinyl, -CO-D-$C_3$-$C_5$-Alkinyl, Trifluormethyl oder durch Cyan, Halogen, $C_1$-$C_4$-Alkoxy oder $C_1$-$C_4$-Alkylthio substituiertes $C_1$-$C_4$-Alkoxy, -D-$C_1$-$C_4$-Alkyl, -D-$C_3$-$C_5$-Alkenyl, -CO-$C_1$-$C_4$-Alkyl, 1,2-Dichlorvinyloxy, -CO-D-$C_1$-$C_4$-Alkyl oder -CO-D-$C_3$-$C_5$-Alkenyl, wobei n eines oder zwei und D eine Sauerstoff-, Schwefel-, -NHoder -N($C_1$-$C_4$-Alkyl)-Brücke bedeuten, steht, dadurch gekennzeichnet, dass man ein Sulfonamid der Formel II

$G$-$SO_2$-$NH_2$ (II)

in Gegenwart einer Base mit einem Chlorameisensäureester der Formel III

Cl-CO-Q-T (III)

oder dass man ein Sulfonylchlorid der Formel IV

$G\text{-}SO_2\text{-}Cl$ (IV)

in Gegenwart einer Base mit einem Urethan der Formel V

$H_2N\text{-}CO\text{-}Q\text{-}T$ (V)

worin G die unter Formel I gegebene Bedeutung hat, und
Q Sauerstoff oder Schwefel und
T $C_1$-$C_4$-Alkyl, Benzyl $C_1$-$C_4$-Halogenalkyl, $C_1$-$C_3$-Cyanalkyl, $C_2$-$C_5$-Alkenyl, oder Alkoxyalkyl oder Alkylthioalkyl mit insgesamt höchstens 5 Kohlenstoffatomen bedeuten, umsetzt und das entstandene Carbamat der Formel VI

$G\text{-}SO_2\text{-}NH\text{-}CO\text{-}Q\text{-}T$ (VI)

durch Umsetzung mit einem Amin der Formel VII

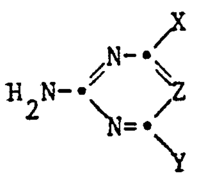

(VII)

worin G, Q, T, X, Y und Z die oben gegebenen Bedeutungen haben, in den Sulfonylharnstoff der Formel I überführt.

2. Verfahren gemäss Anspruch 1, dadurch gekennzeichnet, dass die Umsetzung des Carbamats der Formel VI mit dem Amin der Formel VII bei einer Temperatur von 50 - 220° C erfolgt.

3. Verfahren gemäss Anspruch 2, dadurch gekennzeichnet, dass die Reaktionstemperatur 80 - 160° C beträgt.

4. Verfahren gemäss Anspruch 1, dadurch gekennzeichnet, dass die Umsetzung des Carbamats der Formel VI mit dem Amin der Formel VII in einem inerten Lösungsmittel erfolgt.

5. Verfahren gemäss Anspruch 4, dadurch gekennzeichnet, dass das Lösungsmittel ausgewählt ist aus den Gruppen Kohlenwasserstoffe, Äther, Nitrile, Ketone oder Dimethylsulfoxid.

6. Verfahren gemäss Anspruch 1, dadurch gekennzeichnet, dass die Umsetzung des Sulfonamids der Formel II mit dem Chlorameisensäureester der Formel III oder des Sulfonylchlorids der Formel IV mit dem Urethan der Formel V bei 0° C bis 140° C erfolgt.

7. Verfahren gemäss Anspruch 6, dadurch gekennzeichnet, dass die Reaktionstemperatur 10° C bis 80° C beträgt.

8. Verfahren gemäss Anspruch 1, dadurch gekennzeichnet, dass die Umsetzung des Sulfonamids der Formel II mit dem Chlorameisensaureester der Formel III oder des Sulfonylchlorids der Formel IV mit dem Urethan der Formel V in Gegenwart einer Base aus der Gruppe Carbonate, Hydrogencarbonate, Oxide und tertiäre Amine durchgeführt wird.

9. Verfahren gemäss Anspruch 1, dadurch gekennzeichnet, dass die Umsetzung des Sulfonamids der Formel II mit dem Chloramiesensäureester der Formel III oder des Sulfonylchlorids der Formel IV mit dem Urethan der Formel V in einem inerten Lösungsmittel erfolgt.

10. Verfahren gemäss Anspruch 9, dadurch gekennzeichnet dass das Lösungsmittel ausgewählt ist aus der Gruppe Kohlenwasserstoffe, Äther, Nitrile, Ketone oder Dimethylsulfoxid.

11. Verfahren gemäss Anspruch 1, dadurch gekennzeichnet, dass man ein Sulfonamid der Formel II mit einem Chlorameisensäureester der Formel III, oder dass man ein Sulfonylchlorid der Formel IV in Gegenwart einer Base in einem inerten Lösungsmittel bei 10° bis 80° C mit einem Urethan der Formel V umsetzt und das erhaltene Carbamat der Formel VI zusammen mit einem Amin der Formel VII in einem inerten Lösungsmittel auf 80° bis 160° C erhitzt bis die Abspaltungsreaktion von Alkohol oder Mercaptan abgeschlossen ist und das Produkt isoliert.

12. Sulfonylcarbamate der allgemeinen Formel VIa

$G - SO_2 - NH - CO - Q - T$ (VIa)

worin
Q Sauerstoff oder Schwefel,
T $C_1$-$C_4$-Alkyl, $C_1$-$C_4$-Halogenalkyl, $C_1$-$C_3$-Cyanalkyl, $C_2$-$C_5$-Alkenyl, Benzyl oder Alkoxyalkyl oder Alkylthioalkyl mit insgesamt höchstens 5 Kohlenstoffatomen und
G einen Rest der Formeln

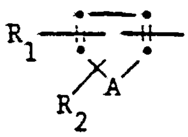 oder 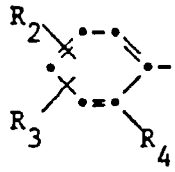 ,

bedeuten, worin

A für Sauerstoff, Schwefel, $-NR_5-$ oder $-C=N-$, wobei $R_5$ Wasserstoff $C_1$-$C_4$-Alkyl oder -CO-$C_1$-$C_4$-Alkyl bedeutet,

$R_1$ für Wasserstoff, $C_1$-$C_4$-Alkyl, Halogen, Nitro, Cyan, $-NH_2$, $-S(O)_n$-$C_1$-$C_4$-Alkyl, $-SO_2$-$C_1$-$C_4$-Alkoxy, $-SO_2$-di-$C_1$-$C_4$-alkylamino, -CHO, $-CONH_2$, -D-$C_3$-$C_5$ -Alkinyl -CO-D-$C_3$-$C_5$ -Alkinyl, -D-$C_1$-$C_4$-Alkyl, -D-$C_3$-$C_5$-Alkenyl, -CO-$C_1$-$C_4$-Alkyl, -CO-D-$C_1$-$C_4$-Alkyl oder -CO-D-$C_3$-$C_5$-Alkenyl, wobei n eines oder zwei und D eine Sauerstoff-, Schwefel-, -NH- oder -N($C_1$-$C_4$-Alkyl)-Brücke bedeuten,

$R_2$ für Wasserstoff, Halogen, $CF_3$, $NO_2$, $C_1$-$C_4$-Alkyl oder $C_1$-$C_4$-Alkoxy und

$R_3$ für Wasserstoff, Fluor oder Chlor stehen und

$R_4$ für Halogen, $C_1$-$C_4$-Alkyl, Nitro, Cyan, $-NH_2$, $-S(O)_n$-$C_1$-$C_4$-Alkyl, $-SO_2$-$C_1$-$C_4$-Alkoxy, $-SO_2$-di-$C_1$-$C_4$-alkylamino, -CHO, $-CONH_2$, -D-$C_3$-$C_5$-Alkinyl, -CO-D-$C_3$-$C_5$-Alkinyl, Trifluormethyl oder durch Cyan, Halogen, $C_1$-$C_4$-Alkoxy oder $C_1$-$C_4$-Alkylthio substituiertes $C_1$-$C_4$-Alkoxy, -D-$C_1$-$C_4$-Alkyl, -D-$C_3$-$C_5$-Alkenyl, -CO-$C_1$-$C_4$-Alkyl, 1,2-Dichlorvinyloxy, -CO-D-$C_1$-$C_4$-Alkyl oder -CO-D-$C_3$-$C_5$-Alkenyl, wobei n eines oder zwei und D eine Sauerstoff-, Schwefel-, -NH- oder -N($C_1$-$C_4$-Alkyl)-Brücke bedeuten, steht, mit der Massgabe, dass

a) der Rest -Q-T nicht Äthoxy oder Butyloxy bedeutet wenn G für den unsubstituierten 2-Thienylrest steht,

b) der Rest -Q-T nicht Methylthio bedeutet, wenn G für den 2-Tolylrest steht und

c) der Rest -Q-T nicht Äthoxy bedeutet, wenn G für den 2-Trifluormethylphenylrest oder den 2-Anisylrest steht.

d) der Rest -Q-T nicht Methoxy bedeutet, wenn G für den 2-Methoxycarbonylphenylrest oder den 2-Äthylthiocarbonylphenylrest steht.

## Claims

1. A process for producing a sulfonylurea of the general formula I

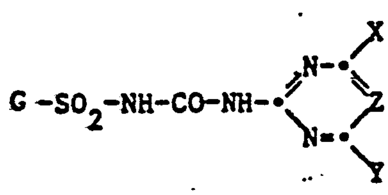 (I)

wherein

Z is an -N= or -CH= radical,

G is a radical of the formula

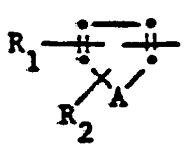 or 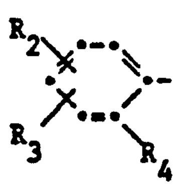 ,

X is $C_1$-$C_4$-alkyl, $C_1$-$C_4$-haloalkyl, $C_1$-$C_4$-alkoxy, $C_1$-$C_4$-alkylthio, halogen, $C_1$-$C_4$-haloalkoxy, $C_1$-$C_4$-alkylamino or di-$C_1$-$C_4$-alkylamino,

Y is $C_1$-$C_4$-alkyl, $C_1$-$C_4$-alkoxy or $C_1$-$C_4$-haloalkoxy,

A is oxygen, sulfur, $-NR_5-$ or $-C=N-$, wherein $R_5$ is hydrogen, $C_1$-$C_4$-alkyl or -CO-$C_1$-$C_4$-alkyl,

$R_1$ is hydrogen, $C_1$-$C_4$-alkyl, halogen, nitro, cyano, $-NH_2$, $-S(O)_n$-$C_1$-$C_4$-alkyl, $-SO_2$-$C_1$-$C_4$-alkoxy, $-SO_2$-di-$C_1$-$C_4$-alkylamino, -CHO, $-CONH_2$, -D-$C_3$-$C_5$-alkynyl, -CO-D-$C_3$-$C_5$-alkynyl, -D-$C_1$-$C_4$-alkyl, -D-$C_3$-$C_5$-alkenyl, -CO-$C_1$-$C_4$-alkyl, -CO-D-$C_1$-$C_4$-alkyl or -CO-D-$C_3$-$C_5$-alkenyl, n being one or two, and D being an oxygen, sulfur, -NH- or -N($C_1$-$C_4$-alkyl)-bridge,

$R_2$ is hydrogen, halogen, $CF_3$, $NO_2$, $C_1$-$C_4$-alkyl or $C_1$-$C_4$-alkoxy,
$R_3$ is hydrogen, fluorine or chlorine, and
$R_4$ is halogen $C_1$-$C_4$-alkyl, nitro, cyano, $-NH_2$, $-S(O)_n$-$C_1$-$C_4$-alkyl, $-SO_2$-$C_1$-$C_4$-alkoxy, $-SO_2$-di-$C_1$-$C_4$-alkylamino, -CHO, $-CONH_2$, -D-$C_3$-$C_5$-alkynyl, -CO-D-$C_3$-$C_5$-alkynyl, trifluoromethyl, or $C_1$-$C_4$-alkoxy substituted by cyano, halogen, $C_1$-$C_4$-alkoxy or $C_1$-$C_4$-alkylthio, or is -D-$C_1$-$C_4$-alkyl, -D-$C_3$-$C_5$-alkenyl, -CO-$C_1$-$C_4$-alkyl, 1,2-dichlorovinyloxy, -CO-D-$C_1$-$C_4$-alkyl or -CO-D-$C_3$-$C_5$-alkenyl, n being one or two, and D being an oxygen, sulfur, -NH- or -N($C_1$-$C_4$-alkyl)- bridge,
which process comprises reacting a sulfonamide of the formula II

$G-SO_2-NH_2$ (II)

in the presence of a base, with a chloroformic acid ester of the formula III

Cl-CO-Q-T (III)

or a sulfonyl chloride of the formula IV

$G-SO_2-Cl$ (IV)

in the presence of a base, with a urethane of the formula V

$H_2N$-CO-Q-T (V)

wherein G has the meaning defined under the formula I,
Q is oxygen or sulfur, and
T is $C_1$-$C_4$-alkyl, benzyl, $C_1$-$C_4$-haloalkyl, $C_1$-$C_3$-cyanoalkyl, $C_2$-$C_5$-alkenyl, or alkoxyalkyl or alkylthioalkyl having a total of at most 5 carbon atoms;
and converting the resulting carbamate of the formula VI

$G-SO_2$-NH-CO-Q-T (VI)

by reaction with an amine of the formula VII

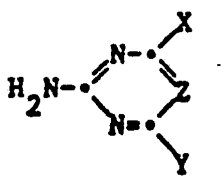

(VII),

wherein G Q T, X, Y and Z have the meanings defined in the foregoing, into the sulfonylurea of the formula I.

2. A process according to Claim 1, wherein the reaction of the carbamate of the formula VI with the amine of the formula VII is performed at a temperature of from 50 - 220° C.

3. A process according to Claim 2, wherein the reaction temperature is from 80 to 160° C.

4. A process according to Claim 1, wherein the reaction of the carbamate of the formula VI with the amine of the formula VII is performed in an inert solvent.

5. A process according to Claim 4, wherein the solvent is selected from the group comprising: hydrocarbons, ethers, nitriles, ketones or dimethyl sulfoxide.

6. A process according to Claim 1, wherein the reaction of the sulfonamide of the formula II with the chloroformic acid ester of the formula III, or of the sulfonyl chloride of the formula IV with the urethane of the formula V, is performed at from 0° to 140° C.

7. A process according to Claim 8, wherein the reaction temperature is from 10° to 80° C.

8. A process according to Claim 1, wherein the reaction of the sulfonamide of the formula II with the chloroformic acid ester of the formula III, or of the sulfonyl chloride of the formula IV with the urethane of the formula V, is performed in the presence of a base from the group comprising: carbonates, hydrogen carbonates, oxides and tertiary amines.

9. A process according to Claim 1, wherein the reaction of the sulfonamide of the formula II with the chloroformic acid ester of the formula III, or of the sulfonyl chloride of the formula IV with the urethane of the formula V, is performed in an inert solvent.

10. A process according to Claim 9, wherein the solvent is selected from the group comprising: hydrocarbons, ethers, nitriles, ketones or dimethyl sulfoxide.

11. A process according to Claim 1, which process comprises reacting a sulfonamide of the formula II with a chloroformic acid ester of the formula III, or reacting a sulfonyl chloride of the formula IV, in the presence of a base and in an inert solvent at from 10° to 80° C, with a urethane of the formula V, and heating the resulting carbamate of the formula VI together with an amine of the formula VII, in an inert solvent at from 80° to 160° C, until the reaction to split off alcohol or mercaptan has finished; and isolating the product.

12. Sulfonylcarbamates of the general formula VIa

G - $SO_2$ - NH - CO - Q - T (VIa)

wherein
Q is oxygen or sulfur,
T is $C_1$-$C_4$-alkyl, $C_1$-$C_4$-haloalkyl, $C_1$-$C_3$-cyanoalkyl, $C_2$-$C_5$-alkenyl, benzyl, or alkoxyalkyl or alkylthioalkyl having a total of at most 5 carbon atoms, and
G is a radical of the formula

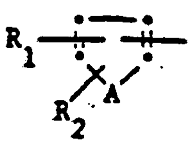

or

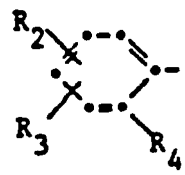

wherein
A is oxygen, sulfur, -$NR_5$- or -C=N-, wherein $R_5$ is hydrogen, $C_1$-$C_4$-alkyl or -CO-$C_1$-$C_4$-alkyl,
$R_1$ is hydrogen, $C_1$-$C_1$-alkyl, halogen, nitro, cyano, -$NH_2$ -$S(O)_n$-$C_1$-$C_4$-alkyl, -$SO_2$-$C_1$-$C_4$-alkoxy, -$SO_2$-di-$C_1$-$C_4$-alkylamino, -CHO, -$CONH_2$, -D-$C_3$-$C_5$-alkynyl, -CO-D-$C_3$-$C_5$-alkynyl, -D-$C_1$-$C_4$-alkyl, -D-$C_3$-$C_5$-alkenyl, -CO-$C_1$-$C_4$-alkyl, -CO-D-$C_1$-$C_4$-alkyl or -CO-D-$C_3$-$C_5$-alkenyl, n being one or two, and D being an oxygen, sulfur, -NH- or -N($C_1$-$C_4$-alkyl)-bridge,
$R_2$ is hydrogen, halogen, $CF_3$, $NO_2$, $C_1$-$C_4$-alkyl or $C_1$-$C_4$-alkoxy,
$R_3$ is hydrogen, fluorine or chlorine, and
$R_4$ is halogen, $C_1$-$C_4$-alkyl, nitro, cyano, -$NH_2$, -$S(O)_n$-$C_1$-$C_4$-alkyl, -$SO_2$-$C_1$-$C_4$-alkoxy, -$SO_2$-di-$C_1$-$C_4$-alkylamino, -CHO, -$CONH_2$, -D-$C_3$-$C_5$-alkynyl, -CO-D-$C_3$-$C_5$-alkynyl, trifluoromethyl, or $C_1$-$C_4$-alkoxy substituted by cyano, halogen, $C_1$-$C_4$-alkoxy or $C_1$-$C_4$-alkylthio, or is -D-$C_1$-$C_4$-alkyl, -D-$C_3$-$C_5$-alkenyl, -CO-$C_1$-$C_4$-alkyl, 1,2-dichlorovinyloxy, -CO-D-$C_1$-$C_4$-alkyl or -CO-D-$C_3$-$C_5$-alkenyl, n being one or two, and D being an oxygen, sulfur, -NH- or -N($C_1$-$C_4$-alkyl)- bridge, with the proviso that,
a) the radical -Q-T is not ethoxy or butoxy when G is the unsubstituted 2-thienyl radical,
b) the radical -Q-T is not methylthio when G is the 2-tolyl radical,
c) the radical -Q-T is not ethoxy when G is the 2-trifluoromethylphenyl radical or the 2-anisyl radical, and
d) the radical -Q-T is not methoxy when G is the 2-methoxycarbonylphenyl radical or the 2-ethylthiocarbonylphenyl radical.

**Revendications**

1. Procédé de préparation d'une sulfonylurée de formule générale I

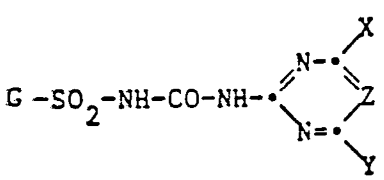

(I)

dans laquelle
Z est un reste -N= ou -CH=,
G est un reste de formules

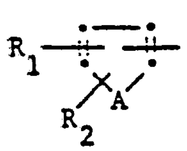

ou

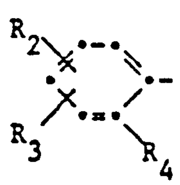

X est un alkyle-$C_1$-$C_4$, halogénoalkyle-$C_1$-$C_4$, alcoxy-$C_1$-$C_4$, alkylthio-$C_1$-$C_4$, halogène, halogénoalcoxy-$C_1$-$C_4$, alkylamino-$C_1$-$C_4$ ou dialkylamino-$C_1$-$C_4$,
Y est un alkyle-$C_1$-$C_4$, alcoxy-$C_1$-$C_4$ ou halogeno-alcoxy-$C_1$-$C_4$,
dans lesquelles
A est l'oxygène, le soufre, -$NR_5$- ou -C=N-, $R_5$ étant l'hydrogène un alkyle-$C_1$-$C_4$ ou un CO-alkyle-$C_1$-$C_4$,

$R_1$ est l'hydrogène, un alkyle-$C_1$-$C_4$, halogène, nitro, cyano, -$NH_2$, -$S(O)_n$-alkyle-$C_1$-$C_4$, -$SO_2$-alcoxy-$C_1$-$C_4$, -$SO_2$-dialkylamino-$C_1$-$C_4$, -CHO, -$CONH_2$, -D-alcynyl-$C_3$-$C_5$, -CO-D-alcynyl-$C_3$-$C_5$, -D-alkyle-$C_1$-$C_4$, -D-alcényle-$C_3$-$C_5$ -CO-alkyle-$C_1$-$C_4$, -CO-D-alkyle-$C_1$-$C_4$ ou -CO-D-alcényle-$C_3$-$C_5$, où n est un ou deux et D est un pont oxygène, soufre, -NH- ou -N(alkyle-$C_1$-$C_4$),

$R_2$ est un hydrogène, halogène, $CF_3$, $NO_2$, alkyle-$C_1$-$C_4$ ou alcoxy-$C_1$-$C_4$ et

$R_3$ est un hydrogène, fluor ou chlore et

$R_4$ est un halogène, alkyle-$C_1$-$C_4$, nitro, cyano, -$NH_2$, -$S(O)_n$-alkyle-$C_1$-$C_4$, -$SO_2$-alcoxy-$C_1$-$C_4$, -$SO_2$-dialkylamino-$C_1$-$C_4$, -CHO, -$CONH_2$, -D-alcynyle-$C_3$-$C_5$, -CO-D-alcynyle-$C_3$-$C_5$ trifluorométhyle ou un alcoxy-$C_1$-$C_4$ substitué par un cyano halogène, alcoxy-$C_1$-$C_4$ ou alkylthio-$C_1$-$C_4$, -D-alkyle-$C_1$-$C_4$, -D-alcényle-$C_3$-$C_5$, -CO-alkyle-$C_1$-$C_4$, 1,2-dichlorovinyloxy -CO-D-alkyle-$C_1$-$C_4$ ou -CO-D-alcényle-$C_3$-$C_5$, où n est un ou deux et D est un pont oxygène, soufre, -NH- ou -N(alkyle-$C_1$-$C_4$), caractérisé en ce qu'on

fait réagir un sulfonamide de formule II

$$G\text{-}SO_2\text{-}NH_2 \qquad (II)$$

en presence d'une base avec un ester d'acide chloroformique de formule III

$$Cl\text{-}CO\text{-}Q\text{-}T \qquad (III)$$

ou on fait réagir un chlorure de sulfonyle de formule IV

$$G\text{-}SO_2\text{-}Cl \qquad (IV)$$

en presence d'une base avec un uréthanne de formule V

$$H_2N\text{-}CO\text{-}Q\text{-}T \qquad (V)$$

dans laquelle G a la signification donnée pour la formule I et

Q est l'oxygène ou le soufre, et

T est un alkyle-$C_1$-$C_4$, benzyle, halogénoalkyle-$C_1$-$C_4$, cyanoalkyle-$C_1$-$C_3$ alcényle-$C_2$-$C_5$ ou alcoxyalkyle ou alkylthioalkyle avec au total 5 atomes de carbone au plus, et on transforme le carbamate formé de formule VI en la sulfonylurée de formule I

$$G\text{-}SO_2\text{-}NH\text{-}CO\text{-}Q\text{-}T \qquad (VI)$$

par réaction avec une amine de formule VII

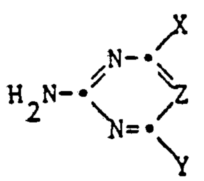

(VII)

dans laquelle G, Q, T, X, Y et Z ont la signification donnée ci-dessus.

2. Procédé selon la revendication 1, caractérisé en ce qu'on fait réagir le carbamate de formule VI avec l'amine de formule VII à une température de 50 - 220° C.

3. Procédé selon la revendication 2, caractérisé en ce que la température de réaction est comprise entre 80 et 160° C.

4. Procédé selon la revendication 1, caractérisé en ce que la réaction du carbamate de formule VI avec l'amine de formule VII a lieu dans un solvant inerte.

5. Procédé selon la revendication 4, caractérisé en ce que le solvant est choisi dans le groupe des hydrocarbures, éthers, nitriles, cétones ou diméthylsulfoxyde.

6. Procédé selon la revendication 1, caractérisé en ce que la réaction du sulfonamide de formule II avec l'ester d'acide chloroformique de formule III, ou la réaction du chlorure de sulfonyle de formule IV avec l'uréthanne de formule V a lieu entre 0° et 140° C.

7. Procédé selon la revendication 6, caractérisé en ce que la température de réaction est comprise entre 10° et 80° C.

8. Procédé selon la revendication 1, caractérisé en ce que la réaction du sulfonamide de formule II avec l'ester de l'acide chloroformique de formule III ou la réaction du chlorure de sulfonyle de formule IV avec l'uréthanne de formule V est réalisée en présence d'une base du groupe des carbonates, hydrogénocarbonates, oxydes et amines tertiaires.

9. Procédé selon la revendication 1, caractérisé en ce que la réaction du sulfonamide de formule II avec l'ester d'acide chloroformique de formule III ou la réaction du chlorure de sulfonyle de formule IV avec l'uréthanne de formule V a lieu dans un solvant inerte.

10. Procédé selon la revendication 9, caractérisé en ce que le solvant est choisi parmi le groupe des hydrocarbures, éthers, nitriles, cetones ou dimethylsulfoxyde.

11. Procédé selon la revendication 1, caractérisé en ce qu'on fait réagir un sulfonamide de formule II avec un ester de l'acide chloroformique de formule III, ou en ce qu'on fait réagir un chlorure de sulfonyle de formule IV en présence d'une base dans un solvant inerte entre 10° et 80°C avec un uréthanne de formule V et qu'on chauffe le carbamate de formule VI obtenu avec une amine de formule VII dans un solvant inerte entre 80° et 160°C, jusqu'à l'accomplissement de l'élimination de l'alcool ou du mercaptan et qu'on isole le produit.

12. Sulfonylcarbamates de formule générale VIa

$$G - SO_2 - NH - CO - Q - T \qquad \text{(VIa)}$$

dans laquelle

Q est l'oxygène ou le soufre,

T est un alkyle-$C_1$-$C_4$, benzyle, halogénoalkyle-$C_1$-$C_4$, cyanoalkyle-$C_1$-$C_3$, alcényle-$C_2$-$C_5$ ou alcoxyalkyle ou alkylthioalkyle avec au total 5 atomes de carbone au plus,

G est un reste de formules

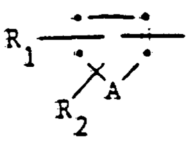

ou

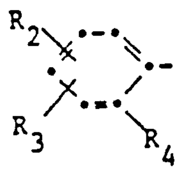

dans lesquelles

A est l'oxygène, le soufre, -$NR_5$- ou -C=N-, $R_5$ étant l'hydrogène, un alkyle-$C_1$-$C_4$ ou un -CO-alkyle-$C_1$-$C_4$,

$R_1$ est l'hydrogène, un alkyle-$C_1$-$C_4$, halogène, nitro, cyano, -$NH_2$, -$S(O)_n$-alkyle-$C_1$-$C_4$, -$SO_2$-alcoxy-$C_1$-$C_4$, -$SO_2$-dialkylamino-$C_1$-$C_4$ -CHO -$CONH_2$, -D-alcynyl-$C_3$-$C_5$ -CO-D-alcynyl-$C_3$-$C_5$ -D-alkyle-$C_1$-$C_4$, -D-alcényle-$C_3$-$C_5$ -CO-alkyle-$C_1$-$C_4$ -CO-D-alkyle-$C_1$-$C_4$ ou -CO-D-alcényle-$C_3$-$C_5$, où n est un ou deux et D est un pont oxygène, soufre, -NH- ou -N(alkyle-$C_1$-$C_4$),

$R_2$ est un hydrogène, halogène $CF_3$, $NO_2$, alkyle-$C_1$-$C_4$ ou alcoxy-$C_1$-$C_4$, et

$R_3$ est un hydrogène, fluor ou chlore, et

$R_4$ est un halogène, alkyle-$C_1$-$C_4$, nitro, cyano, -$NH_2$, -$S(O)_n$-alkyle-$C_1$-$C_4$ -$SO_2$-alcoxy-$C_1$-$C_4$ -$SO_2$-dialkylamino -$C_1$-$C_4$ -CHO, -$CONH_2$, -D-alcynyle-$C_3$-$C_5$, -CO-D-alcynyle-$C_3$-$C_5$, trifluorométhyle ou un alcoxy-$C_1$-$C_4$ substitué par un cyano, halogène, alcoxy-$C_1$-$C_4$ ou alkylthio-$C_1$-$C_4$, -D-alkyle-$C_1$-$C_4$, -D-alcényle-$C_3$-$C_5$, -CO-alkyle-$C_1$-$C_4$, 1,2-dichlorovinyloxy, -CO-D-alkyle-$C_1$-$C_4$ ou -CO-D-alcényle-$C_3$-$C_5$, où n est un ou deux et D est un pont oxygène, soufre, -NH- ou -N(alkyle-$C_1$-$C_4$) avec la condition que

a) le reste -Q-T ne représente pas l'éthoxy ni le butyloxy quand G est le reste non substitué 2-thiényle,

b) le reste -Q-T ne représente pas le méthylthio, quand G est le reste 2-tolyle, et

c) le reste -Q-T ne représente pas l'éthoxy quand G est le reste 2-trifluorométhylphényle ou le reste 2-anisyle,

d) le reste -Q-T ne représente pas le méthoxy, quand G est le reste 2-méthoxycarbonylphényle ou le reste 2-éthylthiocarbonylphényle.